Europäisches Patentamt

European Patent Office

Office européen des brevets

(19)

(11) EP 0 795 317 B1

(12) **EUROPÄISCHE PATENTSCHRIFT**

(45) Veröffentlichungstag und Bekanntmachung des Hinweises auf die Patenterteilung:
**06.06.2001 Patentblatt 2001/23**

(51) Int Cl.⁷: **A61K 7/48**, A61K 7/06

(21) Anmeldenummer: **97103059.8**

(22) Anmeldetag: **26.02.1997**

(54) **Wachskombination und diese enthaltende kosmetische Mittel**

Wax combination and cosmetic compositions containing them

Combinaison de cires et compositions cosmétiques les contenant

(84) Benannte Vertragsstaaten:
**DE ES FR GB IT**

(30) Priorität: **16.03.1996 DE 19610458**
**16.03.1996 DE 19610459**

(43) Veröffentlichungstag der Anmeldung:
**17.09.1997 Patentblatt 1997/38**

(73) Patentinhaber: **Wella Aktiengesellschaft**
**64274 Darmstadt (DE)**

(72) Erfinder:
• **Flemming, Ernst**
**D - 63150 Heusenstamm (DE)**

• **Behner, Ursula**
**D - 64395 Brensbach (DE)**
• **Kischka, Dr. Karl Heinz**
**D - 64293 Darmstadt (DE)**
• **Jacobs, Dr. Anke**
**D - 69151 Neckargemünd (DE)**
• **Bimczok, Dr. Rudolf**
**D- 64342 Seeheim (DE)**
• **Schmich, Bianka**
**D - 64642 Bürstadt (DE)**

(56) Entgegenhaltungen:
**EP-A- 0 569 667**       **WO-A-93/17083**
**FR-A- 2 587 208**

**Beschreibung**

Gegenstand der vorliegenden Erfindung sind eine Wachskombination aus Apfelwachs, Orangenwachs und/ oder Zitronenwachs und Jojobaöl sowie kosmetische Mittel zur Behandlung der Haut oder der Haare, die diese Wachskombination in freier Form oder als einen Wirkstoffkomplex enthält, welcher aus der in Kapseln eingeschlossenen Wachskombination gebildet wird.

In kosmetischen Mitteln können Wachse vielfältige Funktionen erfüllen. Beispielsweise können sie für sich allein zur Abdeckung der Haut verwendet werden oder als Fettkomponente in Emulsionen zur Viskosität und Stabilität des kosmetischen Mittels beitragen. In hautkosmetischen Mitteln eingesetzt, können Wachse der Rückfettung und Hydrophobierung der Haut dienen und in haarkosmetischen Mitteln eine Konditionierung und Pflege des Haares bewirken.

Aufgrund der Herstellungsweise kann man die Wachse in natürliche, chemisch veränderte natürliche und synthetische Wachse einteilen. Zu den für die Kosmetik bedeutsamen Wachsen gehören die Mineralwachse und bestimmte natürliche Wachse tierischer und pflanzlicher Herkunft.

Natürliche und chemisch veränderte natürliche Mineralwachse stammen aus nicht erneuerbaren Rohstoffquellen, bzw. werden aus durch diese erzeugten Rohstoffen hergestellt. Synthetische Mineralwachse sind häufig mit synthesebedingten Verunreinigungen belastet.

Die Verwendung von natürlichen oder chemisch veränderten natürlichen Wachsen tierischer oder pflanzlicher Herkunft vermeidet die zuvor genannten Nachteile der Mineralwachse und kommt zudem den Wünschen weiter Verbraucherkreise nach der Verwendung von natürlichen, ökologisch unbedenklichen, d.h. nachwachsenden Rohstoffen entgegen.

Das bekannteste tierische Wachs ist das Bienenwachs, dessen Verfügbarkeit als Ausscheidungsprodukt der Bienen jedoch begrenzt ist. Darüber hinaus enthält das Bienenwachs hohe Anteile an Estern, was die Stabilität bienenwachshaltiger kosmetischer Mittel nachteilig beeinflussen kann.

Die derzeit am häufigsten in kosmetischen Mitteln eingesetzten, kommerziell erhältlichen pflanzlichen Wachse sind das Candelilla- und das Carnaubawachs. Diese Wachse sind jedoch hart und spröde, haben einen hohen Schmelzpunkt und sind in üblichen kosmetischen Mitteln nur schlecht emulgierbar.

Es wurde nun gefunden, daß eine Wachskombination aus Apfelwachs, Orangen- und/oder Zitronenwachs und Jojobaöl die vorstehend genannten Nachteile nicht aufweist.

Darüber hinaus zeigt die erfindungsgemäße Wachskombination, in kosmetischen Mittel eingesetzt, eine synergistische Steigerung der hautschützenden sowie haut- und haarpflegenden Wirkung gegenüber den einzelnen Komponenten, beispielsweise dem aus der DE-OS 42 06 154 bekannten Apfelwachs.

Weiterhin haben herkömmliche kosmetische Mittel den Nachteil, daß nicht alle Roh- und Wirkstoffe beliebig kombinierbar sind, da es zu unerwünschten Wechselwirkungen zwischen ihnen kommen kann, oder da sie pH-Wertempfindlich sind. Dadurch ist die Auswahl geeigneter Bestandteile und somit auch deren optimale Wirkung stark eingeschränkt.

Es ist bekannt, zur Umgehung dieser Nachteile Mehrkammerverpackungssysteme einzusetzen, die jedoch in der Herstellung aufwendig und teuer sind. Es ist auch bekannt, einzelne Wirkstoffe in Gelatinekapseln einzuschließen, wobei es aber zu Problemen hinsichtlich einer leichten und rückstandslosen Auftragbarkeit und Verteilbarkeit der Wirkstoffe kommen kann.

Es wurde nun gefunden, daß sich die erfindungsgemäße Wachskombination besonders gut in geeigneten Kapseln einschließen läßt, wodurch eine gute Verträglichkeit mit weiteren, in kosmetischen Mitteln einsetzbaren Stoffen erreicht wird und gleichzeitig ein leichtes und rückstandsloses Auftragen und Verteilen der Wachskombination auf der Haut oder dem Haar gewährleistet ist.

Gegenstand der Erfindung ist deshalb eine Wachskombination, bestehend aus

a) 0,01 bis 99 Gewichtsprozent, vorzugsweise 2 bis 70 Gewichtsprozent Apfelwachs,

b) 0,01 bis 99 Gewichtsprozent, vorzugsweise 5 bis 70 Gewichtsprozent Orangen- und/oder Zitronenwachs,

c) 0,01 bis 99 Gewichtsprozent, vorzugsweise 10 bis 70 Gewichtsprozent Jojobaöl.

Ein weiterer Gegenstand der vorliegenden Anmeldung besteht in kosmetischen Mitteln zur Behandlung der Haut oder der Haare, die die vorstehend genannte Wachskombination entweder in freier Form oder als einen Wirkstoffkomplex enthalten, welcher aus der in Kapseln eingeschlossenen Wachskombination gebildet wird.

Für den Wirkstoffkomplex geeignete Kapseln sind aus wasserunlöslichen Polymeren, besonders bevorzugt aus mikrokristalliener Cellulose aufgebaut und enthalten Hydroxypropyl-Methylcellulose und Laktose als Hilfsstoffe. Geeignete Kapseln werden beispielsweise unter der Behandlung Unispheres, insbesondere Unispheres UFW-523 und Unispheres RFW-522 von der Firma Induchem, Dübendorf/Schweiz vertrieben.

In der Literatur sind verschiedene Verfahren zur Herstellung von mit Wirkstoffen beladenen Kapseln beschrieben. Die Verkapselung der erfindungsgemäßen Wachskombination kann nach einem dieser Verfahren erfolgen. Hierzu zählen beispielsweise chemische Verfahren, wobei das zu verkapselnde Produkt in ein flüssiges Medium gegeben wird, in welchem sich um das Produkt herum ein die Wand der Kapseln bildendes Koazervat ausbildet oder die Kapselwand wird durch Kopolymerisation von in dem flüssigem Medium enthaltenen Monomeren gebildet. Es kann auch eines der bekannten chemischen Verfahren zur Verkapselung verwendet werden. Hierzu werden die zu verkapselnden Substanzen beispielsweise in feine Teilchen zentrifugiert und durch einen dünnen Film geleitet, der aus dem die Kapselwand bildenden Material besteht oder die zu verkapselnde Substanz wird im Vakuum zerstäubt, wobei sich auf elektrostatischem Weg ein Überzug absetzt. Ein geeignetes Verfahren ist beispielsweise das in der DE-OS 41 22 591 beschriebene. Besonders bevorzugt ist ein Verkapselungsverfahren, bei welchem sämtliche verwendete Substanzen in Anwesenheit von Wasser zusammengemischt werden und die Kapseln durch das Wirbelschicht-Rotationsverfahren hergestellt werden.

Ein für die erfindungsgemäße Wachskombination geeignetes Apfelwachs wird beispielsweise unter der Handelsbezeichnung Apfelwachs grün 2/080025 von der Firma Dragoco, Holzminden/BRD vertrieben.

Ein für die erfindungsgemäße Wachskombination geeignetes Orangenwachs wird beispielsweise unter der Handelsbezeichnis "Orange peel wax" und ein geeignetes Zitronenwachs unter der Handelsbezeichnung "Lemon peel wax" von der Firma Koster Keunen b.v./Niederlande vertrieben.

Ein für den Einsatz in der erfindungsgemäßen Wachskombination geeignetes Jojobaöl wird beispielsweise unter der Handelsbezeichnung "Jojobaöl" von der Firma Hess GmbH, Stuttgart/BRD vertrieben.

Die erfindungsgemäße Wachskombination wird durch Aufschmelzen der drei Komponenten a), b) und c) bei 50 bis 60°C, ggf. unter Verwendung einer geeigneten Rührvorrichtung und anschließendes Abkühlen hergestellt.

Wird die Wachskombination zur Herstellung des Wirkstoffkomplexes verkapselt, sollen die Kapseln 0,01 bis 30 Gewichtsprozent, vorzugsweise 0,1 bis 20 Gewichtsprozent der Wachskombination, bezogen auf das Kapselgewicht, enthalten. In einem kosmetischen Mittel sollte der Wirkstoffkomplex in einer Menge von 0,01 bis 50 Gewichtsprozent, vorzugsweise in einer Menge von 0,03 bis 20 Gewichtsprozent enthalten sein. Der Wirkstoffkomplex läßt sich leicht und rückstandslos auf die Haut oder das Haar auftragen und verteilen und eignet sich gut zur Herstellung klarer Gele.

Die erfindungsgemäße Wachskombination zeigt ebenso wie der Wirkstoffkomplex eine hautschützende Wirkung und weist einen schwächeren okklusiven Effekt auf als die häufig in kosmetischen Mitteln eingesetzte Vaseline. Sowohl die Wachskombination als auch der Wirkstoffkomplex sind daher als Bestandteil hautschützender und hautpflegender kosmetischer Mittel hervorragend geeignet.

Weiterhin kann die erfindungsgemäße Wachskombination in reiner Form zur Abdeckung und zum Schutz der Haut verwendet werden. Als Bestandteil kosmetischer Haarpflege- und Haarreinigungsmittel verbessert sie ebenso wie der Wirkstoffkomplex die Naß- und Trockenkämmbarkeit sowie den Griff des Haares und schützt das Haar vor den Auswirkungen kosmetischer Behandlungen wie Bleichen, Dauerwellen, Färben und Waschen mit entfettenden Tensiden. Wird die erfindungsgemäße Wachskombination in freier Form in kosmetischen Mitteln verwendet, dann kann ihr Anteil an dem kosmetischen Mittel zwischen 0,03 und 99,8 Gewichtsprozent betragen.

Die erfindungsgemäßen kosmetischen Mittel können beispielsweise als Mittel zur Pflege oder zum Schutz der Haut, zum Beispiel als Tagescreme, Nachtcreme, Hautcreme, Sonnenschutzcreme, Lippenstift, Lippenpflegemittel oder auch als Make up Präperate wie Schminckcreme oder Rouge, als Haar- und/oder Körperreinigungsmittel wie als Shampoo, Duschbad, Schaumbad oder Reinigungslotion eingesetzt werden. Außerdem können sie als Haarbehandlungsmittel wie Haaröl, als Gel oder Haarwachs, als Haarpflegemittel, beispielsweise als Haarkur und Haarspülung oder als Bleichmittel, Dauerverformungsmittel, Fixiermittel, als Haarfärbemittel oder Haartönungsmittel eingesetzt werden.

Wenn es sich bei dem erfindungsgemäßen kosmetischen Mittel um ein emulsionsförmiges Hautschutz- oder Hautpflegemittel handelt, enthält das Mittel bevorzugt 0,03 bis 50 Gewichtsprozent der erfindungsgemäßen Wachskombination in freier Form oder 0,02 bis 15 Gewichtsprozent des Wirkstoffkomplexes. Ein in Form einer Öl-in-Wasser-Emulsion vorliegendes erfindungsgemäßes Hautschutz- oder Hautpflegemittel enthält besonders bevorzugt 0,03 bis 20 Gewichtsprozent der erfindungsgemäßen Wachskombination in freier Form.

Handelt es sich bei dem erfindungsgemäßen kosmetischen Mittel um ein Haar- oder Körperreinigungsmittel oder um ein Haarpflegemittel, dann weist es bevorzugt einen Gehalt von 0,05 bis 5 Gewichtsprozent der Wachskombination in freier Form oder 0,02 bis 4 Gewichtsprozent des Wirkstoffkomplexes auf. In einem derartigen Mittel sind außerdem 0,01 bis 40 Gewichtsprozent mindestens eines anionischen, kationischen, amphoteren und/oder nicht-ionischen Tensids sowie 50 bis 90 Gewichtsprozent Wasser enthalten.

Das erfindungsgemäße Haar- und/oder Körperreinigungsmittel weist einen pH-Wert von 3 bis 8, vorzugsweise 4 bis 7 auf. Erfindungsgemäße Sonnenöle enthalten bevorzugt 0,1 bis 80% der erfindungsgemäßen Wachskombination in freier Form oder 0,01 bis 20 Gewichtsprozent des Wirkstoffkomplexes. Erfindungsgemäße Haaröle, Brillantinen, Haargele und Haarwachse enthalten bevorzugt 1 bis 99,8 Gewichtsprozent der erfindungsgemäßen Wachskombination in freier Form oder 1 bis 19 Gewichtsprozent des Wirkstoffkomplexes.

Erfindungsgemäße Dauerverformungs- und Fixiermittel weisen bevorzugt einen Gehalt von 0,05 bis 5 Gewichtsprozent der erfindungsgemäßen Wachskombination oder des Wirkstoffkomplexes auf und enthalten außerdem 0,05 bis 15 Gewichtsprozent mindestens einer keratinreduzierenden Mercaptoverbindung. Ein derartiges Mittel liegt bevorzugt als wäßrige, alkalisch (pH = 5 bis 10) eingestellte Zubereitung vor, welche als keratinreduzierende Mercaptoverbindung beispielsweise Cystein, Cysteamin, N-Acteyl-L-Cystein, Mercaptocarbonsäuren wie Thioglykolsäure oder Thiomilchsäure oder Salze von Mercaptocarbonsäuren wie Ammonium- und Guanidinsalze der Thioglykolsäure oder Thiomilchsäure enthält.

Die erforderliche Alkalität wird hierbei durch die Zugabe von Ammoniak, organischen Aminen, Ammonium- und Alkalicarbonaten oder -hydrogencarbonaten eingestellt. Es kommt aber auch ein neutral oder sauer (pH = 4,5 bis 7) eingestelltes Haarverformungsmittel in Betracht, das im wäßrigen Medium einen wirksamen Gehalt an Sulfiten oder Mercaptocarbonsäureestern aufweist.

Im ersteren Fall werden vorzugsweise Natrium- oder Ammoniumsulfit oder das Salz der schwefligen Säure mit einem organischen Amin, wie zum Beispiel Monoethanolamin und Guanidin, in einer Konzentration von etwa 2 bis 12 Gewichtsprozent (berechnet als $SO_2$) verwendet. Im letzteren Fall kommen insbesondere Thioglykolsäuremonoglykolester oder -glycerinester in einer Konzentration von etwa 5 bis 50 Gewichtsprozent (entsprechend einem Gehalt an freier Thioglykolsäure von 2 bis 16 Gewichtsprozent) zur Anwendung.

Das erfindungsgemäße Mittel zur dauerhaften Haarverformung kann auch ein Gemisch der vorstehend genannten keratinreduzierenden Verbindungen enthalten.

Nach einer für die dauerhafte Verformung des Haares ausreichenden Einwirkungszeit, welche, je nach Haarbeschaffenheit, dem pH-Wert und der Verformungswirksamkeit des Verformungsmittels sowie in Abhängigkeit von der Anwendungstemperatur, etwa 10 bis 30 Minuten beträgt, wird das Haar mit Wasser gespült und anschließend oxidativ nachbehandelt ("fixiert"). Das Nachbehandlungsmittel wird, je nach Haarfülle, in einer Menge von etwa 50 bis 100 g verwendet.

Für die oxidative Nachbehandlung kann ein erfindungsgemäßes Fixiermittel oder jedes beliebige bisher für eine derartige Behandlung verwendete Fixiermittel verwendet werden. Beispiele für in einem derartigen Fixiermittel verwendbare Oxidationsmittel sind Natrium- und Kaliumbromat, Natriumperborat, Harnstoffperoxid und Hydrogenperoxid.

Die Konzentration des Oxidationsmittels ist in Abhängigkeit von der Anwendungszeit (in der Regel etwa 5 bis 15 Minuten) und der Anwendungstemperatur unterschiedlich. Üblicherweise liegt das Oxidationsmittel in dem wäßrigen Fixiermittel in einer Konzentration von etwa 0,5 bis 10 Gewichtsprozent vor, wobei ein erfindungsgemäßes Fixiermittel zusätzlich 0,05 bis 5 Gewichtsprozent der erfindungsgemäßen Wachskombination enthält.

Sowohl das erfindungsgemäße Mittel zur dauerhaften Haarverformung als auch das erfindungsgemäße Fixiermittel kann in Form einer wäßrigen Lösung oder Emulsion sowie in verdickter Form auf wäßriger Basis, insbesondere als Creme, Gel oder Paste vorliegen. Es ist ebenfalls möglich, dieses Mittel unter Druck in Aerosoldosen abzufüllen und daraus als Schaum zu entnehmen.

Anschließend werden die Wickler entfernt. Falls erforderlich, kann das abgewickelte Haar nun nochmals oxidativ nachbehandelt werden. Sodann wird das Haar mit Wasser gespült, zur Frisur gelegt und schließlich getrocknet.

Das vorstehend beschriebene Verfahren zur dauerhaften Haarverformung ermöglicht eine schonende und gleichmäßige Umformung vom Haaransatz bis zu den Haarspitzen, das Haar zeigt eine hervorragende Naß- und Trockenkämmbarkeit, einen angenehmen Griff und einen ansprechenden Glanz im getrockneten Zustand sowie eine lockere, sprunghafte und gleichmäßige Dauerwellung, insbesondere im Bereich der Haarspitzen.

Ein erfindungsgemäßes Blondiermittel, Oxydationshaarfärbemittel oder Haartönungsmittel enthält bevorzugt 0,05 bis 5 Gewichtsprozent der erfindungsgemäßen Wachskombination oder des Wirkstoffkomplexes.

Ein erfindungsgemäßes Haartönungsmittel enthält zusätzlich zur erfindungsgemäßen Wachskombination 0,05 bis 2,0 Gewichtsprozent mindestens eines direkt auf das Haar aufziehenden Haarfarbstoffs, der beispielsweise aus den folgenden Klassen direkt auf das Haar aufziehender Haarfarbstoffe ausgewählt sein kann: aromatische Nitrofarbstoffe, zum Beispiel 1,4-Diamino-2-nitrobenzol, Azofarbstoffe, zum Beispiel Acid Brown 4 (C. I. 14 805), Anthrachinonfarbstoffe, zum Beispiel Disperse Violet 4 (C. I. 61 105), Triphenylmethanfarbstoffe, zum Beispiel Basic Violet 1 (C. I. 42 535), wobei die Farbstoffe je nach Art ihrer Substituenten sauren, nichtionogenen oder basischen Charakter haben können, und/oder natürliche Haarfarbstoffe, wie zum Beispiel Henna und Reng, der zur Farbentwicklung nicht der Oxidation bedarf.

Das erfindungsgemäße Mittel kann in einer beliebigen für Haut- und Haarbehandlungsmittel geeigneten Zubereitungsform, beispielsweise in Form einer wäßrigalkoholischen oder alkoholischen Lösung, als Emulsion, als Creme oder als Gel vorliegen. Das Mittel kann auch im Gemisch mit einem Treibgas oder mittels einer mechanisch betriebenen Sprühvorrichtung versprüht oder aufgeschäumt werden.

Wenn das erfindungsgemäße Mittel mit Hilfe eines Treibmittels versprüht wird, so enthält es bevorzugt 3 bis 75 Gewichtsprozent des Treibmittels und wird in einem Druckbehälter abgefüllt.

Als Treibmittel sind beispielsweise niedere Alkane, wie zum Beispiel n-Butan, i-Butan und Propan, oder auch deren Gemische mit Dimethylether sowie ferner bei den in Betracht kommenden Drücken gasförmig vorliegende Treibmittel, wie beispielsweise $N_2$, $N_2O$ und $CO_2$ sowie Gemische der vorstehend genannten Treibmittel geeignet.

Unter mechanischen Sprühvorrichtungen sind solche Vorrichtungen zu verstehen, welche das Versprühen einer Flüssigkeit ohne Verwendung eines Treibmittels ermöglichen. Als geeignete mechanische Sprühvorrichtung kann beispielsweise eine Sprühpumpe oder ein mit einem Sprühventil versehener elastischer Behälter, in den das erfindungsgemäße kosmetische Mittel unter Druck abgefüllt wird, wobei sich der elastische Behälter ausdehnt und aus dem das Mittel infolge der Kontraktion des elastischen Behälters bei Öffnen des Sprühventils kontinuierlich abgegeben wird, verwendet werden.

Das erfindungsgemäße kosmetische Mittel kann darüber hinaus für Haar- und Hautbehandlungsmittel übliche Zusatzbestandteile, zum Beispiel Lösungsmittel, wie Wasser und niedere aliphatische Alkohole, beispielsweise Ethanol, Propanol und Isopropanol, oder Glykole wie Glycerin und 1,2-Propylenglykol; Netzmittel oder Emulgatoren aus den Klassen der anionischen, kationischen, amphoteren oder nichtionogenen oberflächenaktiven Tenside, wie Fettalkoholsulfate, Alkylbenzolsulfonate, Alkyltrimethylammoniumsalze, Alkylbetaine, oxethylierte Fettalkohole, oxethylierte Nonylphenole, Fettsäurealkanolamide, oxethylierte Fettsäureester, in einer Menge von 0,1 bis 30 Gewichtsprozent; Parfümöle in einer Menge von 0,5 bis 5,0 Gewichtsprozent; Trübungsmittel, wie zum Beispiel Ethylenglykoldistearat, in einer Menge von etwa 0,5 bis 5,0 Gewichtsprozent; Perlglanzmittel, wie zum Beispiel ein Gemisch aus Fettsäuremonoalkylolamid und Ethylenglykoldistearat, in einer Menge von etwa 1,0 bis 10 Gewichtsprozent; bakterizide und fungizide Wirkstoffe, wie zum Beispiel 2,4,4-Trichlor-2-hydroxydiphenylether oder Methylchlorisothiazolinon, in einer Menge von 0,01 bis 1,0 Gewichtsprozent; Verdickungsmittel, wie beispielsweise Kokosfettsäurediethanolamid, in einer Menge von etwa 0,5 bis 3,0 Gewichtsprozent; Puffersubstanzen wie beispielsweise Natriumcitrat oder Natriumphosphat, in einer Menge von 0,1 bis 1,0 Gewichtsprozent; Lösungsvermittler, wie zum Beispiel ethoxyliertes Rizinusöl, in einer Menge von etwa 0,1 bis 1,0 Gewichtsprozent; Anfärbestoffe wie zum Beispiel Fluorescein-Natriumsalz, in einer Menge von etwa 0,1 , bis 1,0 Gewichtsprozent; Pflegestoffe wie zum Beispiel kationische Harze, Lanolinderivate, in einer Menge von 0,1 bis 5 Gewichtsprozent; Lichtschutzmittel, Feuchthaltemittel, Antioxidantien, Komplexbildner und Antischuppenwirkstoffe in einer Menge von etwa 0,01 bis 0,8 Gewichtsprozent sowie ferner physiologisch verträgliche organische Säuren, wie zum Beispiel Ameisensäure, Glyoxylsäure, Milchsäure, Weinsäure, Zitronensäure, natürliche, modifizierte natürliche oder synthetische Polymere, wie zum Beispiel Schellack, kationische, anionische, nichtionische, amphotere Polymere, Chitosan, Chitin oder Chitosanderivate.

Die nachfolgenden Beispiele sollen den Gegenstand der Erfindung erläutern.

**Beispiele**

**Beispiel 1:** Wachskombination

| | |
|---|---|
| 15 g | Apfelwachs |
| 45 g | Orangenwachs |
| 40 g | Jojobaöl |
| 100 g | |

**Beispiel 2:** Wachskombination

| | |
|---|---|
| 8 g | Apfelwachs |
| 40 g | Orangenwachs |
| 52 g | Jojobaöl |
| 100 g | |

**Beispiel 3:** Wachskombination

| | |
|---|---|
| 20 g | Apfelwachs |
| 30 g | Zitronenwachs |
| 50 g | Jojobaöl |
| 100 g | |

**Beispiel 4:** Wachskombination

| | |
|---|---|
| 12 g | Apfelwachs |
| 48 g | Zitronenwachs |
| 40 g | Jojobaöl |
| 100 g | |

**Beispiel 5:** Hautcreme mit schützender Wirkung

| | |
|---|---|
| 2,00 g | Wachskombination gemäß Beispiel 1 |
| 8,30 g | Glycerinstearat, selbstemulgierend |
| 1,70 g | eines Gemisches aus Glycerylhydroxystearat, Cetylpalmitat, mikrokristallinem Wachs und Trihydroxystearin (Cutina BW, Henkel KGAA) |
| 1,50 g | Stearin |
| 7,50 g | flüssiges Paraffin |
| 0,10 g | p-Hydroxybenzoesäurepropylester |
| 0,30 g | p-Hydroxybenzoesäuremethylester |
| 0,10 g | Allantoin |
| 0,20 g | Parfümöl |
| 78,30 g | Wasser, vollentsalzt |
| 100,00 g | |

**Beispiel 6:** Hautcreme mit schützender Wirkung

| | |
|---|---|
| 4,50 g | Wachskombination gemäß Beispiel 2 |
| 7,00 g | Glycerinstearat, selbstemulgierend |
| 1,40 g | Stearin |
| 7,60 g | flüssiges Paraffin |
| 0,10 g | p-Hydroxybenzoesäurepropylester |
| 0,30 g | p-Hydroxybenzoesäuremethylester |
| 0,10 g | Allantoin 0,20 g Parfümöl |
| 78,80 g | Wasser, vollentsalzt |
| 100,00 g | |

**Beispiel 7:** Lippenpflegestift

| | |
|---|---|
| 26,00 g | Rizinusöl |
| 4,00 g | Babassuöl |
| 19,00 g | Mineralöl |
| 28,00 g | mikrokristallines Wachs (Lunacera ® M) |
| 14,00 g | Decyloleat |
| 7,00 g | Wachskombination gemäß Beispiel 2 |
| 2,00 g | Wasser, vollentsalzt |
| 100,00 g | |

**Beispiel 8:** Sonnenschutzcreme (Öl-in-Wasser-Emulsion)

| | |
|---|---|
| 7,00 g | Glycerylstearat |
| 2,50 g | Cetylstearylalkohol |
| 1,80 g | Cetylstearylalkohol mit 20 Mol Ethylenoxid oxethyliert |
| 1,40 g | Cetylstearylalkohol mit 12 Mol Ethylenoxid oxethyliert |
| 3,20 g | Wachskombination gemäß Beispiel 1 |
| 6,50 g | gemischte Triester des Glycerins mit Caprinsäure und Caprylsäure |
| 7,50 g | Gemisch der Ester der Caprinsäure und Caprylsäure mit Kokosnußalkohol |
| 6,20 g | Dibutyladipat |
| 3,80 g | Octyldodecanol |
| 2,50 g | Dimethylpolysiloxan |
| 3,80 g | 4-Methoxy-zimtsäure-2-ethylhexylester |
| 2,70 g | Glycerin |
| 51,10 g | Wasser, vollentsalzt |
| 100,00 g | |

**Beispiel 9:** Shampoo

| | |
|---|---|
| 2,744 g | Natriumlaurylethersulfat |
| 0,029 g | Benzoesäure |
| 1,500 g | Kokosfettsäureamidopropylbetain |
| 2,000 g | Ethylenglykoldistearat |
| 0,400 g | Wachskombination gemäß Beispiel 3 |
| 0,400 g | Parfümöl |
| 3,300 g | Natriumchlorid |
| 89,627 g | Wasser, vollentsalzt |
| 100,00 g | |

**Beispiel 10:** Haarpflegespülung

| | |
|---|---|
| 3,50 g | Cetylstearylalkohol |
| 0,50 g | Wachskombination gemäß Beispiel 4 |
| 0,60 g | Cetyltrimethylammoniumchlorid |
| 0,40 g | Zitronensäure |
| 0,40 g | Parfümöl |
| 94,60 g | Wasser, vollentsalzt |

(fortgesetzt)

| 100,00 g | |
|---|---|

**Beispiel 11:** Haarpflegespülung

| 2,50 g | Cetylstearylalkohol |
|---|---|
| 0,80 g | Laurylalkoholdiglykolether |
| 1,10 g | Vaseline |
| 1,20 g | Wachskombination gemäß Beispiel 4 |
| 0,40 g | Cetylstearylsulfat-Natriumsalz |
| 5,00 g | Betainmonohydrat |
| 0,20 g | Benzoesäure |
| 0,80 g | Glyoxylsäure |
| 0,20 g | Parfümöl |
| 87,80 g | Wasser, vollentsalzt |
| 100,00 g | |

**Beispiel 12:** Haarpflegeschaum, auszuspülen

| 0,40 g | Wachskombination gemäß Beispiel 4 |
|---|---|
| 10,00 g | Ethanol |
| 0,30 g | Polyoxyethylenglykolderivat des Rizinusöls (CTFA: PEG-35 caster oil) |
| 0,30 g | Polyethylenglykol(4)laurylether |
| 0,10 g | Cetyltrimethylammoniumchlorid |
| 0,07 g | Isopropanol |
| 1,40 g | Vinylimidazoliniummethochlorid/Vinylpyrrolidon-Copolymer (CTFA: Polyquaternum-16) |
| 0,30 g | Parfümöl |
| 87,13 g | Wasser |
| 100,00 g | |

96 g des vorstehenden Haarpflegemittels werden mit 4 g eines Gemisches aus 50 Gewichtsprozent Propan und 50 Gewichtsprozent n-Butan in einen Aerosolbehälter abgefüllt. Der erfindungsgemäße Pflegeschaum wird beim Versprühen in Schaumform dem Behälter entnommen.

**Beispiel 13:** Haarpflegemittel, nicht auszuspülen

| 0,100 g | Wachskombination gemäß Beispiel 3 |
|---|---|
| 0,112 g | Isopropanol |
| 0,600 g | Distearyldimethylammoniumchlorid |
| 0,200 g | Glycerinmonodistearat |
| 0,150 g | Polyoxyethylen-(45)-polyoxypropylen-(33)monobutylether |
| 0,100 g | Stearylalkohol |
| 0,090 g | Stearylbetain |
| 0,100 g | p-Hydroxybenzoesäuremethylester |
| 0,840 g | Ethanol |
| 97,708 g | Wasser |
| 100,00 g | |

**Beispiel 14:** Haarstylinggel

| | |
|---|---|
| 0,10 g | Wachskombination gemäß Beispiel 3 |
| 3,00 g | Polyvinylpyrrolidon |
| 0,50 g | Polyacrylsäure (CTFA: Carbomer) |
| 12,00 g | Ethanol |
| 0,10 g | Ammoniak |
| 84,30 g | Wasser |
| 100,00 g | |

**Beispiel 15:** Haarspray

| | |
|---|---|
| 0,20 g | Wachskombination gemäß Beispiel 1 |
| 5,00 g | Polyvinylpyrrolidon/Vinylacetat-Copolymer |
| 0,20 g | Parfümöl |
| 60,00 g | n-Butan |
| 34,60 g | Ethanol |
| 100,00 g | |

**Beispiel 16:** Versprühbares Haarpflegemittel, nicht auszuspülen

| | |
|---|---|
| 0,30 g | Wachskombination gemäß Beispiel 2 |
| 0,10 g | Keratinhydrolysat |
| 0,40 g | Cetyltrimethylammoniumchlorid |
| 0,28 g | Isopropanol |
| 0,33 g | Kokosfettsäureamidopropylbetain |
| 0,90 g | Polyvinylpyrrolidon |
| 0,20 g | Zitronensäure |
| 97,49 g | Wasser |
| 100,00 g | |

**Beispiel 17:** Versprühbares Haarpflegemittel, nicht auszuspülen

| | |
|---|---|
| 0,20 g | Wachskombination gemäß Beispiel 2 |
| 0,80 g | Polyvinylpyrrolidon |
| 0,32 g | Vinylimidazoliniummethochlorid/Vinylpyrrolidon-Copolymer |
| 0,21 g | Kokosfettsäureamidopropylbetain |
| 0,05 g | mit 7 Mol Polyethylenglykol derivatisiertes hydriertes Rizinusöl (CTFA: PEG-7-hydrogenated caster oil) |
| 0,18 g | mit 40 Mol Polyethylenglykol derivatisiertes hydriertes Rizinusöl (CTFA: PEG-40-hydrogenated caster oil) |
| 0,20 g | Keratinhydrolisat |
| 12,00 g | Ethanol |
| 0,20 g | Glyoxylsäure |
| 85,84 g | Wasser |
| 100,00 g | |

**Beispiel 18:** Dauerverformungsmittel

| | |
|---|---|
| 0,15 g | Wachskombination gemäß Beispiel 2 |
| 8,82 g | Thioglykolsäure |
| 0,90 g | mit 35 Mol Polyethylenglykol derivatisiertes Rizinusöl (CTFA: PEG-35-caster oil) |
| 0,90 g | Polyethylenglykol-(23)-nonylphenolether |
| 0,90 g | Poly(diallyldimethylammoniumchlorid) |
| 0,28 g | Styrol/Polyvinylpyrrolidon-Copolymer |
| 0,15 g | Ammoniak |
| 3,50 g | Ammoniumhydrogencarbonat |
| 0,50 g | Parfümöl |
| 83,90 g | Wasser |
| 100,00 g | |

**Beispiel 19:** Fixiermittel für die Dauerverformung (Neutralisationsmittel)

| | |
|---|---|
| 0,200 g | Wachskombination gemäß Beispiel 1 |
| 2,610 g | Cetylstearylalkohol |
| 0,290 g | Natriumlaurylsulfat |
| 0,400 g | Lanolinalkohol |
| 1,900 g | Wasserstoffperoxyd |
| 0,085 g | Phosphorsäure |
| 0,100 g | Salicylsäure |
| 0,300 g | Parfümöl |
| 94,115 g | Wasser |
| 100,00 g | |

**Beispiel 20:** Haartönungsschaum

| | |
|---|---|
| 0,2000 g | Wachskombination gemäß Beispiel 3 |
| 4,5000 g | Cetylstearylalkohol |
| 1,750 g | Cetyltrimethylammoniumchloridd |
| 4,2250 g | Isopropanol |
| 0,0025 g | Basic Violet 14 (C. I. Nr. 42510) |
| 89,322 g | Wasser |
| 100,00 g | |

94,00 g des vorstehenden Haartönungsschaumes werden mit 6 g eines Gemisches aus 50 Gewichtsprozent Propan, 40 Gewichtsprozent n-Butan und 10 Gewichtsprozent Dimethylether in einen Aerosolbehälter abgefüllt. Das erfindungsgemäße Haartönungsmittel wird beim Versprühen dem Behälter in Schaumform entnommen.

**Beispiel 21:** Oxydatives Haarfärbemittel (dunkelbraun)

| | |
|---|---|
| 0,60 g | Wachskombination gemäß Beispiel 2 |
| 17,10 g | Cetylstearylalkohol |

(fortgesetzt)

| | |
|---|---|
| 1,90 g | Natriumlaurylsulfat |
| 2,10 g | Lanolinalkohol |
| 6,10 g | Glycerylstearat, selbstemulgierend |
| 0,40 g | Natriumsalz des Kokosfettsäureesters der Isoethionsäure (CTFA: Sodium cocoylisoethionate) |
| 1,40 g | Natriumlaurylethersulfat |
| 0,06 g | p-Aminophenolhydrochlorid |
| 0,65 g | P-Toluylendiaminsulfat |
| 0,26 g | Resorcin |
| 0,60 g | Natriumsulfit |
| 0,30 g | Ethylendiamintetraessigsäure |
| 6,00 g | Isopropanol |
| 0,30 g | Parfümöl |
| 1,40 g | Ammoniak |
| 60,83 g | Wasser |
| 100,00 g | |

Vor der Anwendung werden 50 g des vorstehenden Mittels mit 50 ml Wasserstoffperoxid (6%ige Lösung) vermischt.

**Beispiel 22:** Wirkstoffkomplex

| | |
|---|---|
| 15 g | Apfelwachs |
| 45 g | Orangenwachs |
| 40 g | Jojobaöl |
| 100 g, | |

eingeschlossen in 1.000 g Kapseln aus Cellulose, Hydroxypropyl-methylcellulose und Lactose (Unispheres der Firma Induchem/Dübendorf, Schweiz)

**Beispiel 23:** Wirkstoffkomplex

| | |
|---|---|
| 8 g | Apfelwachs |
| 40 g | Orangenwachs |
| 52 g | Jojobaöl |
| 100 g, | |

eingeschlossen in 500 g Kapseln aus Cellulose, Hydroxypropyl-methylcellulose und Lactose (Unispheres der Firma Induchem/Dübendorf, Schweiz).

**Beispiel 24:** Wirkstoffkomplex

| | |
|---|---|
| 20 g | Apfelwachs |
| 30 g | Zitronenwachs |
| 50 g | Jojobaöl |
| 100 g, | |

eingeschlossen in 770 g Kapseln aus Cellulose, Hydroxypropyl-methylcellulose und Lactose (Unispheres der Firma Induchem/Dübendorf, Schweiz).

**Beispiel 25:** Wirkstoffkomplex

| | |
|---|---|
| 12 g | Apfelwachs |
| 48 g | Zitronenwachs |
| 40 g | Jojobaöl |
| 100 g, | |

eingeschlossen in 556 g Kapseln aus Cellulose, Hydroxypropyl-methylcellulose und Lactose (Unispheres der Firma Induchem/Dübendorf, Schweiz).

**Beispiel 26:** Hautcreme mit schützender Wirkung

| | |
|---|---|
| 10,00 g | Wirkstoffkomplex gemäß Beispiel 22 |
| 8,30 g | Glycerinstearat, selbstemulgierend |
| 1,70 g | eines Gemisches aus Glycerylhydroxystearat, Cetylpalmitat, mikrokristallinem Wachs und Trihydroxystearin |
| 1,50 g | Stearin |
| 7,50 g | flüssiges Paraffin |
| 0,10 g | p-Hydroxybenzoesäurepropylester |
| 0,30 g | p-Hydroxybenzoesäuremethylester |
| 0,10 g | Allantoin |
| 0,20 g | Parfümöl |
| 70,30 g | Wasser, vollentsalzt |
| 100,00 g | |

**Beispiel 27:** Hautcreme mit schützender Wirkung

| | |
|---|---|
| 12,00 g | Wirkstoffkomplex gemäß Beispiel 25 |
| 7,00 g | Glycerinstearat, selbstemulgierend |
| 1,40 g | Stearin |
| 7,60 g | flüssiges Paraffin |
| 0,10 g | p-Hydroxybenzoesäurepropylester |
| 0,30 g | p-Hydroxybenzoesäuremethylester |
| 0,10 g | Allantoin |
| 0,20 g | Parfümöl |
| 71,30 g | Wasser, vollentsalzt |
| 100,00 g | |

**Beispiel 28:** Sonnenschutzcreme (Öl-in-Wasser-Emulsion)

| | |
|---|---|
| 7,00 g | Glycerylstearat |
| 2,50 g | Cetylstearylalkohol |
| 1,80 g | Cetylstearylalkohol mit 20 Mol Ethylenoxid oxethyliert |

(fortgesetzt)

| | |
|---|---|
| 1,40 g | Cetylstearylalkohol mit 12 Mol Ethylenoxid oxethyliert |
| 8,00 g | Wirkstoffkomplex gemäß Beispiel 22 |
| 6,50 g | gemischte Triester des Glycerins mit Caprinsäure und Caprylsäure |
| 7,50 g | Gemisch der Ester der Caprinsäure und Caprylsäure mit Kokosnußalkohol |
| 6,20 g | Dibutyladipat |
| 3,80 g | Octyldodecanol |
| 2,50 g | Dimethylpolysiloxan |
| 3,80 g | 4-Methoxy-zimtsäure-2-ethylhexylester |
| 2,70 g | Glycerin |
| 46,30 g | Wasser, vollentsalzt |
| 100,00 g | |

**Beispiel 29:** Shampoo

| | |
|---|---|
| 2,74 g | Natriumlaurylethersulfat |
| 0,03 g | Benzoesäure |
| 1,50 g | Kokosfettsäureamidopropylbetain |
| 2,00 g | Ethylenglykoldistearat |
| 0,80 g | Wirkstoffkomplex gemäß Beispiel 24 |
| 0,40 g | Parfümöl |
| 3,30 g | Natriumchlorid |
| 89,23 g | Wasser, vollentsalzt |
| 100,00 g | |

**Beispiel 30:** Haarpflegespülung

| | |
|---|---|
| 3,50 g | Cetylstearylalkohol |
| 0,70 g | Wirkstoffkomplex gemäß Beispiel 25 |
| 0,60 g | Cetyltrimethylammoniumchlorid |
| 0,40 g | Zitronensäure |
| 0,40 g | Parfümöl |
| 94,40 g | Wasser, vollentsalzt |
| 100,00 g | |

**Beispiel 31:** Haarpflegespülung

| | |
|---|---|
| 2,50 g | Cetylstearylalkohol |
| 0,80 g | Laurylalkoholdiglykolether |
| 1,10 g | Vaseline |
| 1,20 g | Wirkstoffkomplex gemäß Beispiel 25 |
| 0,40 g | Cetylstearylsulfat-Natriumsalz |
| 5,00 g | Betainmonohydrat |
| 0,20 g | Benzoesäure |
| 0,80 g | Glyoxylsäure |

(fortgesetzt)

| | |
|---|---|
| 0,20 g | Parfümöl |
| 87,80 g | Wasser, vollentsalzt |
| 100,00 g | |

**Beispiel 32:** nicht auszuspülendes Pflegemittel

| | |
|---|---|
| 0,50 g | Wirkstoffkomplex gemäß Beispiel 23 |
| 0,50 g | Polyacrylsäure |
| 0,40 g | 2-Amino-2-methyl-1-propanol |
| 0,10 g | Vinylpyrrolidon/Dimethylaminoethyl-methacrylat Copolymer |
| 0,20 g | Hydriertes Rizinusöl, ethoxyliert mit 45 Mol Ethylenoxid |
| 0,25 g | D-Panthenol |
| 0,05 g | Laurylpolyglucose |
| 12,00 g | Ethanol |
| 86,00 g | Wasser |
| 100,00 g | |

**Beispiel 33:** Haarstylinggel

| | |
|---|---|
| 0,80 g | Wirkstoffkomplex gemäß Beispiel 24 |
| 3,00 g | Polyvinylpyrrolidon |
| 0,50 g | Polyacrylsäure (CTFA: Carbomer) |
| 12,00 g | Ethanol |
| 0,20 g | Parfümöl |
| 0,10 g | Ammoniak |
| 83,40 g | Wasser |
| 100,00 g | |

**Beispiel 34:** Haarpflegemittel, nicht auszuspülen

| | |
|---|---|
| 0,70 g | Wirkstoffkomplex gemäß Beispiel 23 |
| 0,10 g | Keratinhydrolysat |
| 0,40 g | Cetyltrimethylammoniumchlorid |
| 0,28 g | Isopropanol |
| 0,33 g | Kokosfettsäureamidopropylbetain |
| 0,90 g | Polyvinylpyrrolidon |
| 0,20 g | Zitronensäure |
| 97,09 g | Wasser |
| 100,00 g | |

**Beispiel 35:** Haarpflegemittel, nicht auszuspülen

| | |
|---|---|
| 0,80 g | Wirkstoffkomplex gemäß Beispiel 22 |
| 0,80 g | Polyvinylpyrrolidon |
| 0,32 g | Vinylimidazoliniummethochlorid/Vinylpyrrolidon-Copolymer |
| 0,21 g | Kokosfettsäureamidopropylbetain |
| 0,20 g | Keratinhydrolysat |
| 12,00 g | Ethanol |
| 0,20 g | Glyoxylsäure |
| 85,47 g | Wasser |
| 100,00 g | |

**Beispiel 36:** Dauerverformungsmittel

| | |
|---|---|
| 0,70 g | Wirkstoffkomplex gemäß Beispiel 23 |
| 8,82 g | Thioglykolsäure |
| 0,90 g | mit 35 Mol Polyethylenglykol derivatisiertes Rizinusöl (CTFA: PEG-35-caster oil) |
| 0,90 g | Polyethylenglykol-(23)-nonylphenolether |
| 0,90 g | Poly(diallyldimethylammoniumchlorid) |
| 0,28 g | Styrol/Polyvinylpyrrolidon-Copolymer |
| 0,15 g | Ammoniak |
| 3,50 g | Ammoniumhydrogencarbonat |
| 0,50 g | Parfümöl |
| 83,35 g | Wasser |
| 100,00 g | |

**Beispiel 37:** Fixiermittel für die Dauerverformung (Neutralisationsmittel)

| | |
|---|---|
| 1,100 g | Wirkstoffkomplex gemäß Beispiel 22 |
| 2,610 g | Cetylstearylalkohol |
| 0,290 g | Natriumlaurylsulfat |
| 0,400 g | Lanolinalkohol |
| 1,900 g | Wasserstoffperoxyd |
| 0,085 g | Phosphorsäure |
| 0,100 g | Salicylsäure |
| 0,300 g | Parfümöl |
| 93,215 g | Wasser |
| 100,000 g | |

**Beispiel 38:** Haartönungsmittel

| | |
|---|---|
| 0,6000 g | Wirkstoffkomplex gemäß Beispiel 24 |
| 4,5000 g | Cetylstearylalkohol |
| 1,750 g | Cetyltrimethylammoniumchlorid |
| 4,2250 g | Isopropanol |
| 0,0025 g | Basic Violet 14 (C. I. Nr. 42510) |
| 88,9225 g | Wasser |
| 100,0000 g | |

**Beispiel 39:** Oxydatives Haarfärbemittel (dunkelbraun)

| | |
|---|---|
| 0,80 g | Wirkstoffkomplex gemäß Beispiel 23 |
| 17,10 g | Cetylstearylalkohol |
| 1,90 g | Natriumlaurylsulfat |
| 2,10 g | Lanolinalkohol |
| 6,10 g | Glycerylstearat, selbstemulgierend |
| 0,40 g | Natriumsalz des Kokosfettsäureesters der Isethionsäure (CTFA: Sodium cocoylisethionate) |
| 1,40 g | Natriumlaurylethersulfat |
| 1,40 g | Ammoniak |
| 0,06 g | p-Aminophenolhydrochlorid |
| 0,65 g | P-Toluylendiaminsulfat |
| 0,26 g | Resorcin |
| 0,60 g | Natriumsulfit |
| 0,30 g | Ethylendiamintetraessigsäure |
| 6,00 g | Isopropanol |
| 0,30 g | Parfümöl |
| 60,63 g | Wasser |
| 100,00 g | |

Vor der Anwendung werden 50 g des vorstehenden Mittels mit 50 ml Wasserstoffperoxid (6%-ige Lösung) vermischt. Soweit nicht anderes vermerkt ist, sollen alle Prozentangaben Gewichtsprozent bedeuten.

**Patentansprüche**

1. Wachskombination bestehend aus

   a) 0,01 bis 99 Gewichtsprozent, vorzugsweise 2 bis 70 Gewichtsprozent Apfelwachs,

   b) 0,01 bis 99 Gewichtsprozent, vorzugsweise 5 bis 70 Gewichtsprozent Orangen- und/oder Zitronenwachs und

   c) 0,01 bis 99 Gewichtsprozent, vorzugsweise 10 bis 70 Gewichtsprozent Jojobaöl.

2. Kosmetisches Mittel zur Behandlung der Haut und/oder der Haare, **dadurch gekennzeichnet,** daß es die Wachskombination von Anspruch 1 in freier Form oder als einen Wirkstoffkomplex enthält, welcher aus der in Kapseln eingeschlossenen Wachskombination gebildet wird.

3. Mittel nach Anspruch 2, **dadurch gekennzeichnet,** daß der Wirkstoffkomplex aus Kapseln besteht, die 0,01 bis 30 Gewichtsprozent, vorzugsweise 0,1 bis 20 Gewichtsprozent der Wachskombination von Anspruch 1 enthalten, welche von einem wasserunlöslichen Polymeren, vorzugsweise mikrokristalliner Cellulose mit Hydroxypropylcellulose und Lactose als Hilfsstoffen umhüllt ist.

4. Mittel nach Anspruch 3, **dadurch gekennzeichnet,** daß der Wirkstoffkomplex in einer Menge von 0,01 bis 50 Gewichtsprozent, vorzugsweise in einer Menge von 0,03 bis 20 Gewichtsprozent in dem kosmetischen Mittel enthalten ist.

5. Mittel nach Anspruch 2, **dadurch gekennzeichnet,** daß es ein emulsionsförmiges Hautschutz- oder Hautpflegemittel ist, das 0,03 bis 50 Gewichtsprozent der Wachskombination in freier Form oder 0,02 bis 15 Gewichtsprozent des Wirkstoffkomplexes enthält.

6. Mittel nach Anspruch 2, **dadurch gekennzeichnet,** daß es ein Haarpflegemittel ist, das 0,05 bis 5 Gewichtsprozent der Wachskombination in freier Form oder 0,02 bis 4 Gewichtsprozent des Wirkstoffkomplexes enthält.

**7.** Mittel nach Anspruch 2, **dadurch gekennzeichnet,** daß es ein Haar- und/oder Körperreinigungsmittel ist, das

0,05 bis 5 Gewichtsprozent der Wachskombination in freier Form oder 0,02 bis 4 Gewichtsprozent des Wirkstoffkomplexes sowie

0,01 bis 40 Gewichtsprozent mindestens eines anionischen, kationischen, amphoteren oder nicht-ionischen Tensids oder eines Gemisches von zwei oder mehreren dieser Tenside und

50 bis 90 Gewichtsprozent Wasser

enthält.

**8.** Mittel nach Anspruch 2, **dadurch gekennzeichnet,** daß es ein Dauerverformungsmittel ist, das

0,05 bis 5 Gewichtsprozent der Wachskombination oder des Wirkstoffkomplexes und

0,5 bis 15 Gewichtsprozent mindestens einer keratinreduzierenden Mercaptoverbindung

enthält.

**9.** Mittel nach Anspruch 2, **dadurch gekennzeichnet,** daß es ein Blondiermittel, ein Oxydationshaarfärbemittel oder ein Haartönungsmittel ist, das 0,05 bis 5 Gewichtsprozent der Wachskombination oder des Wirkstoffkomplexes enthält.

**10.** Mittel nach Anspruch 9, **dadurch gekennzeichnet,** daß es ein Haartönungsmittel ist und zusätzlich 0,05 bis 2 Gewichtsprozent eines direkt auf das Haar aufziehenden und/oder eines natürlichen Haarfarbstoffes enthält.

**Claims**

**1.** Wax combination consisting of

a) 0.01 to 99 per cent by weight, preferably 2 to 70 per cent by weight, of apple wax,
b) 0.01 to 99 per cent by weight, preferably 5 to 70 per cent by weight, of orange and/or lemon wax and
c) 0.01 to 99 per cent by weight, preferably 10 to 70 per cent by weight, of jojoba oil.

**2.** Cosmetic composition for treating the skin and/or hair, characterized in that it comprises the wax combination of Claim 1 in free form or as an active ingredient complex which is formed from the wax combination enclosed in capsules.

**3.** Composition according to Claim 2, characterized in that the active ingredient complex consists of capsules which comprise 0.01 to 30 per cent by weight, preferably 0.1 to 20 per cent by weight, of the wax combination of Claim 1, which is surrounded by a water-insoluble polymer, preferably microcrystalline cellulose with hydroxypropylcellulose and lactose as auxiliaries.

**4.** Composition according to Claim 3, characterized in that the active ingredient complex is present in the cosmetic composition in an amount of from 0.01 to 50 per cent by weight, preferably in an amount of from 0.03 to 20 per cent by weight.

**5.** Composition according to Claim 2, characterized in that it is an emulsion-like skin protection or skin care composition which comprises 0.03 to 50 per cent by weight of the wax combination in free form or 0.02 to 15 per cent by weight of the active ingredient complex.

**6.** Composition according to Claim 2, characterized in that it is a hair care composition which comprises 0.05 to 5 per cent by weight of the wax combination in free form or 0.02 to 4 per cent by weight of the active ingredient complex.

**7.** Composition according to Claim 2, characterized in that it is a hair and/or body cleansing composition which comprises

0.05 to 5 per cent by weight of the wax combination in free form or 0.02 to 4 per cent by weight to the active ingredient complex and
0.01 to 40 per cent by weight of at least one anionic, cationic, amphoteric or nonionic surfactant or a mixture of two or more of these surfactants and
50 to 90 per cent by weight of water.

**8.** Composition according to Claim 2, characterized in that it is a permanent shaping composition which comprises

0.05 to 5 per cent by weight of the wax combination or of the active ingredient complex and
0.5 to 15 per cent by weight of at least one keratin-reducing mercapto compound.

**9.** Composition according to Claim 2, characterized in that it is a bleach, an oxidation hair colorant or a hair tint which comprises 0.05 to 5 per cent by weight of the wax combination or of the active ingredient complex.

**10.** Composition according to Claim 9, characterized in that it is a hair tint and additionally comprises 0.05 to 2 per cent by weight of a hair dye which acts directly on the hair and/or of a natural hair dye.


**Revendications**

**1.** Association de cires constituée de :

a) de 0,01 à 99 pour cent en poids, de préférence de 2 à 70 pour cent en poids de cire de pomme,
b) de 0,01 à 99 pour cent en poids, de préférence de 5 à 70 pour cent en poids de cire d'orange et/ou de citron et
c) de 0,01 à 99 pour cent en poids, de préférence de 10 à 70 pour cent en poids d'huile de jojoba.

**2.** Composition cosmétique destinée au traitement de la peau et/ou des cheveux, caractérisée en ce qu'elle comprend l'association de cires selon la revendication 1 sous forme libre ou en tant que complexe actif qui est formé à partir de l'association de cires contenue dans des capsules.

**3.** Composition selon la revendication 2, caractérisée en ce que le complexe actif est constitué de capsules comprenant de 0,01 à 30 pour cent en poids, de préférence de 0,1 à 20 pour cent en poids de l'association de cires selon la revendication 1, qui est recouverte d'un polymère insoluble dans l'eau, de préférence de cellulose microcristalline avec de l'hydroxypropylcellulose et du lactose en tant qu'auxiliaires.

**4.** Composition selon la revendication 3, caractérisée en ce que le complexe actif est présent en une quantité de 0,01 à 50 pour cent en poids, de préférence en une quantité de 0,03 à 20 pour cent en poids dans la composition cosmétique.

**5.** Composition selon la revendication 2, caractérisée en ce qu'il s'agit d'une composition protectrice pour la peau ou d'une composition de soins de la peau émulsifiante, comprenant de 0,03 à 50 pour cent en poids de l'association de cires sous forme libre ou de 0,02 à 15 pour cent en poids du complexe actif.

**6.** Composition selon la revendication 2, caractérisée en ce qu'il s'agit d'une composition de soins des cheveux comprenant de 0,05 à 5 pour cent en poids de l'association de cires sous forme libre ou de 0,02 à 4 pour cent en poids du complexe actif.

**7.** Composition selon la revendication 2, caractérisée en ce qu'il s'agit d'une composition nettoyante pour les cheveux et/ou le corps comprenant

de 0,05 à 5 pour cent en poids de l'association de cires sous forme libre ou de 0,02 à 4 pour cent en poids du complexe actif, ainsi que
de 0,01 à 40 pour cent en poids d'au moins un tensioactif anionique, cationique, amphotère ou non ionique ou d'un mélange de deux ou plus de ces tensioactifs, et de 50 à 90 pour cent en poids d'eau.

**8.** Composition selon la revendication 2, caractérisée en ce qu'il s'agit d'une composition d'ondulation permanente comprenant

de 0,05 à 5 pour cent en poids de l'association de cires ou du complexe actif, et
de 0,5 à 15 pour cent en poids d'au moins un composé mercapto réduisant la kératine.

9. Composition selon la revendication 2, caractérisée en ce qu'il s'agit d'une composition décolorante, d'une composition colorante d'oxydation pour les cheveux ou d'une composition de nuançage des cheveux comprenant de 0,05 à 5 pour cent en poids de l'association de cires ou du complexe actif.

10. Composition selon la revendication 9, caractérisée en ce qu'il s'agit d'une composition de nuançage des cheveux comprenant en outre de 0,05 à 2 pour cent en poids d'un colorant capillaire s'absorbant directement sur les cheveux et/ou d'un colorant capillaire naturel.